# EUROPEAN PATENT SPECIFICATION

(11) **EP 2 588 112 B1**
(45) Date of publication and mention of the grant of the patent: **27.09.2017**
(21) Application number: 11810107.0
(22) Date of filing: 28.06.2011
(51) Int. Cl.: A61K 31/5513, A61K 31/551, A61K 31/55, A61K 31/519, A61K 31/4178, A61P 25/28, A61P 25/00

(54) **METHODS AND COMPOSITIONS FOR TREATMENT OF MULTIPLE SCLEROSIS**
VERFAHREN UND ZUSAMMENSETZUNGEN ZUR BEHANDLUNG VON MULTIPLER SKLEROSE
PROCÉDÉS ET COMPOSITIONS POUR LE TRAITEMENT DE LA SCLÉROSE EN PLAQUES

(30) Priority: 30.06.2010 US 360020 P
(43) Date of publication of application: 08.05.2013
(60) Divisional application: 17186290.7
(73) Proprietor: Victoria Link Ltd, Wellington 6140 (NZ)
(72) Inventor: LAFLAMME, Anne, Camille, Wellington 6011 (NZ); CONNOR, Bronwen, Jane, North Shore City 0626 (NZ); O'SULLIVAN, David, Palmerston North 4410 (NZ)
(74) Representative: Elsworth, Jon David
(86) International application number: PCT/US2011/042244
(87) International publication number: WO 2012/012147

(56) References cited:
- WO-A1-2008/052354
- US-A1- 2008 146 541
- US-A1- 2008 286 234
- US-A1- 2008 305 140
- US-A1- 2010 119 624
- JOSIASSEN ET AL: "Clozapine augmentation with risperidone in refractory schizophrenia", SCHIZOPHRENIA RESEARCH, ELSEVIER, NETHERLANDS, vol. 60, no. 1, 15 March 2003 (2003-03-15) , page 288, XP005398000, ISSN: 0920-9964, DOI: 10.1016/S0920-9964(03)80474-7
- HOUTHOOFD S A M K ET AL: "Cognitive and psychomotor effects of risperidone in schizophrenia and schizoaffective disorder", CLINICAL THERAPEUTICS, EXCERPTA MEDICA, PRINCETON, NJ, US, vol. 30, no. 9, September 2008 (2008-09), pages 1565-1589, XP025496090, ISSN: 0149-2918, DOI: 10.1016/J.CLINTHERA.2008.09.014 [retrieved on 2008-09-01]
- R. M. BILDER ET AL: "Neurocognitive Effects of Clozapine, Olanzapine, Risperidone, and Haloperidol in Patients With Chronic Schizophrenia or Schizoaffective Disorder", AMERICAN JOURNAL OF PSYCHIATRY, vol. 159, no. 6, June 2002 (2002-06), pages 1018-1028, XP055085825, ISSN: 0002-953X, DOI: 10.1176/appi.ajp.159.6.1018
- DATABASE MEDLINE [Online] US NATIONAL LIBRARY OF MEDICINE (NLM), BETHESDA, MD, US; 1996, GALLHOFER B ET AL: "Cognitive dysfunction in schizophrenia: comparison of treatment with atypical antipsychotic agents and conventional neuroleptic drugs.", XP002715630, Database accession no. NLM8792116 & GALLHOFER B ET AL: "Cognitive dysfunction in schizophrenia: comparison of treatment with atypical antipsychotic agents and conventional neuroleptic drugs.", EUROPEAN NEUROPSYCHOPHARMACOLOGY : THE JOURNAL OF THE EUROPEAN COLLEGE OF NEUROPSYCHOPHARMACOLOGY 1996, vol. 6 Suppl 2, 1996, pages S13-S20, ISSN: 0924-977X
- FRIEDMAN J H ET AL: "ATYPICAL ANTIPSYCHOTICS IN THE TREATMENT OF DRUG-INDUCED PSYCHOSIS IN PARKINSON'S DISEASE", MOVEMENT DISORDERS, RAVEN PRESS, NEW YORK, NY, US, vol. 15, no. 2, March 2000 (2000-03), pages 201-211, XP002950202, ISSN: 0885-3185, DOI: 10.1002/1531-8257(200003)15:2<201::AID-MDS 1001>3.0.CO;2-D
- VOKAS C S ET AL: "Incidence of risperidone induced incontinence", SCHIZOPHRENIA RESEARCH, ELSEVIER, NETHERLANDS, vol. 24, no. 1-2, January 1997 (1997-01), page 267, XP027460374, ISSN: 0920-9964, DOI: 10.1016/S0920-9964(97)82769-7 [retrieved on 1997-01-01]
- GEORGOPOULOU EKATERINI ET AL: "Psychosis in a multiple sclerosis patient and antipsychotic treatment", ANNALS OF GENERAL PSYCHIATRY, BIOMED CENTRAL, LONDON, GB, vol. 7, no. Suppl 1, 17 April 2008 (2008-04-17), page S254, XP021038775, ISSN: 1744-859X
- CHONG S A ET AL: "Clozapine treatment of psychosis associated with multiple sclerosis.", CANADIAN JOURNAL OF PSYCHIATRY. REVUE CANADIENNE DE PSYCHIATRIE FEB 1997, vol. 42, no. 1, February 1997 (1997-02), pages 90-91, XP009173840, ISSN: 0706-7437
- DATABASE MEDLINE [Online] US NATIONAL LIBRARY OF MEDICINE (NLM), BETHESDA, MD, US; November 2008 (2008-11), HUSSAIN A ET AL: "Risperidone depot in the treatment of psychosis associated with multiple sclerosis -- a case report.", XP002715631, Database accession no. NLM18308807 & HUSSAIN A ET AL: "Risperidone depot in the treatment of psychosis associated with multiple sclerosis -- a case report.", JOURNAL OF PSYCHOPHARMACOLOGY (OXFORD, ENGLAND) NOV 2008, vol. 22, no. 8, November 2008 (2008-11), pages 925-926, ISSN: 0269-8811
- SIDOTI ET AL: "Multiple sclerosis and Capgras' syndrome", CLINICAL NEUROLOGY AND NEUROSURGERY, ELSEVIER, AMSTERDAM, NL, vol. 109, no. 9, 16 September 2007 (2007-09-16), pages 786-787, XP022251065, ISSN: 0303-8467, DOI: 10.1016/J.CLINEURO.2007.05.022
- VIRLEY ET AL: "Developing Therapeutics for the Treatment of Multiple Sclerosis", JOURNAL OF THE AMERICAN SOCIETY FOR EXPERIMENTALNEUROTHERAPEUTICS, XX, XX, vol. 2, no. 4, October 2005 (2005-10), pages 638-649, XP027825679, ISSN: 1545-5343 [retrieved on 2005-10-01]
- COHEN ET AL: "The future of multiple sclerosis treatment", JOURNAL OF NEUROLOGICAL SCIENCES, ELSEVIER SCIENTIFIC PUBLISHING CO, AMSTERDAM, NL, vol. 277, February 2009 (2009-02), pages S55-S61, XP025912540, ISSN: 0022-510X, DOI: 10.1016/S0022-510X(09)70015-2 [retrieved on 2009-02-01]
- JÜRGEN HAAS ET AL: "Reduced suppressive effect of CD4+CD25high regulatory T cells on the T cell immune response against myelin oligodendrocyte glycoprotein in patients with multiple sclerosis", EUROPEAN JOURNAL OF IMMUNOLOGY, vol. 35, no. 11, November 2005 (2005-11), pages 3343-3352, XP055085663, ISSN: 0014-2980, DOI: 10.1002/eji.200526065
- J. E. LEYSEN ET AL.: 'Risperidone: a novel antipsychotic with balanced serotonin-dopamine antagonism, receptor occupancy profile, and pharmacological activity' JOURNAL OF CLINICAL PSYCHIATRY vol. 55, May 1994, pages SUPPL. 5 - 12
- G. OKUGAWA ET AL.: 'Randomized clinical comparison of perospirone and risperidone in patients with schizophrenia: Kansai psychiatric multicenter study' PSYCHIATRY AND CLINICAL NEUROSCIENCES vol. 63, 2009, pages 322 - 328
- E. ZEINSTRA ET AL.: 'Aminergic receptors in astrogliotic plaques from patients with multiple sclerosis' NEUROSCIENCES LETTERS vol. 331, 2002, pages 87 - 90

## Description

### BACKGROUND

### 1. Field

The present disclosure is directed to pharmaceutical formulations for use in the treatment of at least one symptom of multiple sclerosis using the antipsychotic drug clozapine.

### 2. Description of the Related Art

Multiple sclerosis (abbreviated MS, also known as disseminated sclerosis or encephalomyelitis disseminata) is a disease in which the fatty myelin sheaths around the axons of the brain and spinal cord are damaged, leading to demyelination and scarring as well as a broad spectrum of signs and symptoms. Onset of MS usually occurs in young adults, and it is more common in females. Prevalence ranges between 2 and 150 per 100,000.

Multiple sclerosis is known for affecting the ability of nerve cells in the brain and spinal cord to communicate with each other. Typically, nerve cells communicate by sending electrical signals called action potentials down long fibers called axons, which are wrapped in an insulating substance called myelin. An autoimmune reaction in MS is thought to result in loss of myelin function. When myelin is lost, the axons can no longer effectively conduct signals. The name multiple sclerosis refers to scars (scleroses-better known as plaques or lesions) in the white matter of the brain and spinal cord, which is mainly composed of myelin. Although much is known about the mechanisms involved in the disease process, the cause remains unknown.

A person with MS can suffer almost any neurological symptom or sign, including changes in sensation such as loss of sensitivity or tingling, pricking or numbness (hypoesthesia and paraesthesia), muscle weakness, clonus, muscle spasms, or difficulty in moving; difficulties with coordination and balance (ataxia); problems in speech (dysarthria) or swallowing (dysphagia), visual problems (nystagmus, optic neuritis including phosphenes, or diplopia), fatigue, acute or chronic pain, and bladder and bowel difficulties.

It is theorized that multiple sclerosis may be caused by genetics, infections or environmental factors. Genetically speaking, MS is not considered a hereditary disease, however, a number of genetic variations have been shown to increase the risk of developing the disease. The risk of acquiring MS is higher in relatives of a person with the disease than in the general population. The disease has an overall familial recurrence rate of 20%. Certain genes have been linked with multiple sclerosis. Differences in the human leukocyte antigen (HLA) system-a group of genes in chromosome 6 that serves as the major histocompatibility complex (MHC) in humans appear to increase the probability acquiring multiple sclerosis. The most consistent finding is the association between multiple sclerosis and alleles of the MHC defined as DR15 and DQ6.

The acquisition of multiple sclerosis may include environmental factors. For example, multiple sclerosis appears to be more common with decreased sunlight exposure as hypothesized in a study by Marrie, Lancet Neurol., 3:709-718, 2004. Additionally, evidence for viruses as a cause includes the presence of oligoclonal bands in the brain and cerebrospinal fluid of most patients, the association of several viruses with human demyelination encephalomyelitis and the induction of demyelination in animals through viral infection. Ewing and Bernard, Immunol. Cell Biol., 76:47-54, 1998 have demonstrated that a mouse model of multiple sclerosis, known as experimental autoimmune encephalomyelitis has proven to be vital in understanding the pathogenesis of multiple sclerosis and in testing the efficacy of currently available MS treatments.

Multiple sclerosis can present as almost any neurological symptom and often progresses to physical and cognitive disability. MS may take several forms, with new symptoms occurring either in discrete attacks (relapsing forms) or slowly accumulating over time (progressive forms). Between attacks, symptoms may go away completely, but permanent neurological problems often occur, especially as the disease advances.

Multiple sclerosis currently has no known cure. However, there are some treatment options available. Typically treatments attempt to return function after an attack, prevent new attacks, and prevent disability. Unfortunately many MS medications can have adverse effects or can be poorly tolerated. The prognosis is difficult to predict; it depends on the subtype of the disease, the individual patient's disease characteristics, the initial symptoms and the degree of disability the person experiences as time advances.

Although there is no known cure for multiple sclerosis, several therapies have proven helpful. During symptomatic attacks, administration of high doses of intravenous corticosteroids, such as methylprednisolone, is the routine therapy for acute relapses. Although generally effective in the short term for relieving symptoms, corticosteroid treatments do not appear to have a significant impact on long-term recovery. Currently, five disease-modifying treatments have been approved by regulatory agencies of different countries for MS. Interferon beta-1a (trade names Avonex, CinnoVex, ReciGen and Rebif) and interferon beta-1b (U.S. trade name Betaseron, in Europe and Japan Betaferon). A third medication is glatiramer acetate (Copaxone), a non-interferon, non-steroidal immunomodulator. A fourth medication, mitoxantrone, is an immunosuppressant also used in cancer chemotherapy. The fifth is natalizumab (marketed as Tysabri). The interferons and glatiramer acetate are delivered by frequent injections, varying from once-per-day for glatiramer acetate to once-per-week (but intra-muscular) for Avonex. Natalizumab and mitoxantrone are given by IV infusion at monthly intervals. Thus, more available treatment options are desirable.

### SUMMARY

The present disclosure is directed to the use of clozapine, a drug with an affinity for both serotonergic 5-HT₂ and dopaminergic D₂ receptors, in pharmaceutical formulations for use in reducing at least one symptom of multiple sclerosis, wherein the dose of clozapine is in the range of 1 mg/kg/body to 5 mg/kg/body.

In certain instances, the drug may be used to treat a subject, which may be a human subject or a mammal subject diagnosed with multiple sclerosis or a symptom or symptoms of multiple sclerosis.

In certain instances, the drug may be used on a subject diagnosed with multiple sclerosis or exhibiting one or more symptoms of multiple sclerosis.

In certain embodiments, the formulation may also include risperidone and/or paliperidone.

In certain embodiments, symptoms of multiple sclerosis include one or more of the following: incontinence, loss of memory, paralysis, loss of coordination, fatigue, pain, optic neuritis, spasticity, transverse myelitis, tremor or a combination thereof.

In certain embodiments the drug or drugs are formulated in a pharmaceutically acceptable formulation.

In certain embodiments the drug or drugs are administered to the subject orally, epidurally, transdermally, subcutaneously, intravenously, intramuscularly, intrathecally, intraperitoneally or via inhalation.

The foregoing and other objects, features and advantages of the present disclosure will become more readily apparent from the following detailed description of exemplary embodiments as disclosed herein.

### BRIEF DESCRIPTION OF THE DRAWINGS

The following figures form part of the present specification and are included to further demonstrate certain aspects of the present disclosure. The disclosure may be better understood by reference to one or more of the figures in combination with the detailed description of specific embodiments presented herein. Embodiments of the present disclosure are described, by way of example only, with reference to the attached figures, wherein:
FIG. 1A illustrates a comparative exemplary treatment of C57BL/6 mice with risperidone (administered in drinking water; 3 mg/kg/day) following immunization to induce EAE with MOG (myelin oligodendrocyte glycoprotein) peptide in complete Freund's adjuvant. Disease score is based on the progressive ascending paralysis that characterizes EAE in this animal model where 0 = normal and 5 = complete body paralysis (i.e. moribund).
FIG. 1B illustrates a comparative exemplary treatment of C57BL/6 mice with risperidone (administered in drinking water; 3 mg/kg/day) following immunization to induce EAE. The data is from 4 separate and independent experiments.
FIG. 1C illustrates a comparative exemplary reduction in the "area under the curve" (AUC), which is an indication of the reduction in total disease burden through administration of risperidone. AUC was measured from days 0-26 and the results are from 4 separate and independent experiments.
FIG. 1D illustrates a comparative exemplary dose-response with 3 mg/kg/day having a significant protective effect (p < 0.0001 by 2-way ANOVA).
FIG. 1E illustrates a comparative exemplary incidence graph.
FIG. 2A illustrates an exemplary dose-dependent reduction in disease severity through administration of clozapine (administered in drinking water; 15 or 30 mg/kg/day) in C57BL/6 mice immunized to induce EAE.
FIG. 2B illustrates an exemplary reduction in AUC through administration of clozapine.
FIG. 3A illustrates that neither risperidone (comparative) nor clozapine cause a reduction in total splenocyte numbers (i.e. do not cause leukocyte death) in unimmunized or immunized C57BL/6 mice treated with risperidone (3 mg/kg/day) or clozapine (30 mg/kg/day).
FIG. 3B illustrates that risperidone (comparative) alters the neutrophil profile of lymphoid tissue in C57BL/6 mice 40 days after immunization with MOG peptide antigen.
FIG. 3C illustrates that risperidone (comparative) does not alter the CD4 helper T cell profile of lymphoid tissue in C57BL/6 mice 40 days after immunization with MOG peptide antigen.
FIG. 3D illustrates that risperidone (comparative) alters the splenic T regulatory cell profile of lymphoid tissue in C57BL/6 mice 40 days after immunization with MOG peptide antigen.
FIG. 4A illustrates exemplary antigen specific IL-17 production (pg/ml) in C57BL/6 mice, which were treated with risperidone (comparative), clozapine, or vehicle alone, and immunized to induce EAE 21 days earlier. Shown are the mean and standard error of the mean (n=5 mice per group).
FIG. 4B illustrates exemplary polyclonal IL-17 production (pg/ml) in C57BL/6 mice, which were treated with risperidone (comparative), clozapine, or vehicle alone, and immunized to induce EAE 21 days earlier. Shown are the mean and standard error of the mean (n=5 mice per group).
FIG. 4C illustrates exemplary antigen specific interferon γ production (pg/ml) in C57BL/6 mice, which were treated with risperidone (comparative), clozapine, or vehicle alone, and immunized to induce EAE 21 days earlier. Shown are the mean and standard error of the mean (n=5 mice per group).
FIG. 4D illustrates exemplary polyclonal interferon γ production (pg/ml) in C57BL/6 mice, which were treated with risperidone (comparative), clozapine, or vehicle alone, and immunized to induce EAE 21 days earlier. Shown are the mean and standard error of the mean (n=5 mice per group).
FIG. 4E illustrates exemplary antigen specific IL-10 production (pg/ml) in C57BL/6 mice, which were treated with risperidone (comparative), clozapine, or vehicle alone, and immunized to induce EAE 21 days earlier. Shown are the mean and standard error of the mean (n=5 mice per group).
FIG. 4F illustrates exemplary polyclonal IL-10 production (pg/ml) in C57BL/6 mice, which were treated with risperidone (comparative), clozapine, or vehicle alone, and immunized to induce EAE 21 days earlier. Shown are the mean and standard error of the mean (n=5 mice per group).
FIG. 5A illustrates comparative exemplary antigen specific (L-17 production (pg/ml) in C57BL/6 mice, which were treated with risperidone or vehicle alone, and immunized to induce EAE 40 days earlier. Shown are the mean and standard error of the mean (n=5-10 mice per group).
FIG. 5B illustrates comparative exemplary antigen specific interferon γ production (pg/ml) in C57BL/6 mice, which were treated with risperidone or vehicle alone, and immunized to induce EAE 40 days earlier. Shown are the mean and standard error of the mean (n=5-10 mice per group).
FIG. 5C illustrates comparative exemplary antigen specific IL-10 production (pg/ml) in C57BL/6 mice, which were treated with risperidone or vehicle alone, and immunized to induce EAE 40 days earlier. Shown are the mean and standard error of the mean (n=5-10 mice per group).
FIG. 5D illustrates comparative exemplary T cell proliferation in counts per minute in immunized and normal C57BL/6 mice, which were treated with risperidone or vehicle alone. Shown are the mean and standard error of the mean (n=5-10 mice per group).
FIG. 6A illustrates reduced weight lost in risperidone-treated mice (comparative) after immunization, even when sick with EAE.
FIG. 6B illustrates reduced weight lost in clozapine-treated mice after immunization, even when sick with EAE.
FIG. 7A illustrates that treatment of normal and immunized mice with risperidone (comparative) but not clozapine leads to hyperprolactinemia.
FIG. 7B illustrates that the level of prolactin in the serum does not correlate to EAE disease score in risperidone (comparative) or clozapine treated or vehicle treated mice.
FIG. 8A illustrates treatment of BALB/c mice with risperidone (comparative) following immunization to induce EAE with PLP (proteolipid protein) peptide in complete Freund's adjuvant. This model is more resistant to EAE but develops brain lesions more similar to MS patients than the C57BL/6 model. Shown are the disease scores of sick mice only (n = 8/ group) combined from 2 independent experiments; there is no difference in % incidence (73% for each group).
FIG. 8B illustrates that in the absence of IL-4Rα, risperidone (comparative) is not effective at reducing disease in BALB/c mice suggesting that this receptor which is involved in Th2 response is essential for risperidone-mediated protection. Shown are the disease scores of sick mice only (n = 8-9/ group) combined from 2 independent experiments; there is no difference in % incidence.
FIG. 8C illustrates that although risperidone (comparative) does not protect IL-4Rα-deficient BALB/c mice, the plasma hyperprolactinemia is similar to WT BALB/c.
FIG. 9A illustrates comparative exemplary Iba-1 expression on microglia in the cerebellum of mice. Shown are images from the cerebellum of unimmunized or immunized mice that have been treated with risperidone or vehicle. Tissue samples were taken at peak disease (15 days post immunization). Shown are the scores for microglia reactivity (Iba score) and disease.
FIG. 9B illustrates that risperidone (comparative) reduces microglial reactivity (measured by Iba-1 expression) in the cerebellum of immunized mice. One group (risperidone d11) was immunized but only received risperidone treatment starting at day 11 (i.e. after disease onset). Scores for Iba-1 reactivity were assessed blinded and derived from a combined score for Iba intensity (0-3) and # Iba+ foci (0-3).
FIG. 9C illustrates that microglial reactivity in the cerebellum (i.e. Iba score) correlates to disease score in untreated but not risperidone-treated (comparative), immunized mice.
FIG. 9D illustrates that risperidone (comparative) reduces microglial reactivity (measured by Iba-1 expression) in the brains of immunized mice. Scores for Iba-1 reactivity were assessed blinded and derived from a combined score (0-6) for Iba intensity (0-3) and number of Iba+ foci (0-3). Scores for the following brain regions were combined: cerebellum, brain stem, hippocampus, and olfactory bulb (0-24).
FIG. 9E illustrates that microglial reactivity (i.e. Iba-1 score) throughout the brain (cerebellum, hippocampus, brain stem, and olfactory bulb) correlates to disease score in untreated and in risperidone-treated (comparative), immunized mice although the Iba and disease scores are reduced in the risperidone-treated mice.
FIG. 9F illustrates that clozapine reduces microglial reactivity (measured by Iba-1 expression) in the brains of immunized mice. Scores for Iba-1 reactivity in the cerebellum were assessed blinded and derived from a combined score (0-6) for Iba intensity (0-3) and number of Iba+ foci (0-3).
FIG. 10A illustrates that exemplary doses of 20 µg/ml risperidone (comparative) or 10 µg/ml clozapine do not alter bone-marrow-derived macrophage viability even in the presence of the microbial product lipopolysaccharide (LPS) as measured by MTT dye reduction assay.
FIG. 10B illustrates that when bone marrow-derived macrophages are incubated with LPS to induce pro-inflammatory cytokine secretion, the addition of 20 µg/ml risperidone (comparative) or 10 µg/ml clozapine reduces IL-12 production.
FIG. 10C illustrates that when bone marrow-derived macrophages are incubated with LPS to induce pro-inflammatory cytokine secretion, the addition of 20 µg/ml risperidone (comparative) or 10 µg/ml clozapine reduces NO production.
FIG. 10D illustrates that when bone marrow-derived macrophages are incubated with LPS to induce pro-inflammatory cytokine secretion, the addition of risperidone (comparative) enhances IL-10 production.
FIG. 11A illustrates that both risperidone (comparative) and clozapine cause a reduction in MHC class II expression (change in geometric mean fluorescence intensity) on splenic macrophages (F4/80⁺ cells) in immunized C57BL/6 mice treated with risperidone (3 mg/kg/day) or clozapine (30 mg/kg/day) compared to immunized, vehicle-treated mice and assessed by flow cytometric analysis during peak disease.
FIG. 11B illustrates that risperidone (comparative) causes a reduction in CD40 expression (change in geometric mean fluorescence intensity) on splenic macrophages (F4/80⁺ cells) in immunized C57BL/6 mice treated with risperidone (3 mg/kg/day) compared to vehicle-treated, immunized mice and assessed by flow cytometric analysis during peak disease.
FIG. 11C illustrates that risperidone (comparative) does not alter the number of CD40⁺ splenic macrophages expression (expressed as % of total splenic F4/80⁺ cells) in immunized C57BL/6 mice treated with risperidone (3 mg/kg/day) compared to vehicle-treated, immunized mice and assessed by flow cytometric analysis during peak disease.

### DETAILED DESCRIPTION

It will be appreciated that for simplicity and clarity of illustration, where considered appropriate, reference numerals may be repeated among the figures to indicate corresponding or analogous elements. In addition, numerous specific details are set forth in order to provide a thorough understanding of the example embodiments described herein. However, it will be understood by those of ordinary skill in the art that the example embodiments described herein may be practiced without these specific details. In other instances, methods, procedures and components have not been described in detail so as not to obscure the embodiments described herein.

The present disclosure is directed to pharmaceutical formulations for use in the treatment of at least one symptom of multiple sclerosis, wherein the formulation comprises the drug clozapine and the dose of clozapine is in the range of 1 mg/kg/body to 5 mg/kg/body.

In examplary embodiments, a subject in need of treatment, such as a mammal, and in specific embodiments a human, is administered the pharmaceutical formulation. In such embodiments, the pharmaceutical formulation may also include risperidone and/or paliperidone.

In specific embodiments, the subject in need of treatment is a subject exhibiting one or more signs of multiple sclerosis. In such embodiments, symptoms may include one or more of the following: 1) changes in sensation such as loss of sensitivity or tingling, 2) pricking or numbness (hypoesthesia and paraesthesia), 3) muscle weakness, clonus, muscle spasms, or difficulty in moving; 4) difficulties with coordination and balance (ataxia); 5) problems in speech (dysarthria) or swallowing (dysphagia), 6) visual problems (nystagmus, optic neuritis including phosphenes, or diplopia), 7) fatigue, acute or chronic pain, 8) bladder and bowel difficulties, and 9) cognitive impairment.

In certain embodiments, a subject, such as a mammal or a human may be first assessed for a symptom associated with multiple sclerosis and then treated with the pharmaceutical formulation. In certain further embodiments, the drug is administered in combination with other drugs. For example, risperidone and clozapine may be administered or risperidone, paliperidone and clozapine may be administered or clozapine and paliperidone may be administered. In exemplary embodiments wherein more than one drug is administered to a subject, the drugs may be administered at the same time or at different times. In exemplary embodiments wherein the drugs are administered at the same time, the drugs may be formulated together, such as a capsule or intravenous formulation which contains two or more of the drugs.

### a. Atypical Antipsychotics

Risperidone is an atypical antipsychotic that has been shown to be effective in the treatment schizophrenia, schizoaffective disorder, bipolar disorder, and irritability in children with autism. Risperidone has also been used off-label for the treatment of anxiety disorders, such as obsessive-compulsive disorder; severe, treatment-resistant depression with or without psychotic features; Tourette syndrome; disruptive behavior disorders in children; and eating disorders. Risperidone has also been reported to successfully treat the symptoms of phencyclidine (PCP) psychosis due to acute intoxication and chronic use. The drug was developed by Janssen-Cilag and first released in 1994. It is sold under the trade name Risperdal in the Netherlands, United States, Canada, Australia, United Kingdom, Portugal, Spain, Turkey, New Zealand and several other countries, Risperdal or Ridal in New Zealand, Sizodon or Riscalin in India, Rispolept in Eastern Europe, and Russia, and Belivon, or Rispen elsewhere.

Pharmacologically, risperidone has a chemical designation of 3-[2-[4-(6-fluoro-1,2-benzisoxazol-3-yl)-1-piperidinyl]ethyl]-6,7,8,9-tetrahydro-2-methyl-4H-pyrido[1,2-a]pyrimidin-4-one and is a selective monoaminergic antagonist belonging to benzisoxazole derivatives. Risperidone has a high affinity for both serotonergic 5-HT₂ and dopaminergic D₂ receptors. It also binds to α1-adrenergic receptors and with lower affinity to H₁-histaminergic and α2-adrenergic receptors. Risperidone has no affinity for cholinergic receptors. Risperidone contains the functional groups of benzisoxazole and piperidine as part of its molecular structure. As a potent D2 antagonist, risperidone is indicated for use in the treatment of schizophrenia, causing lower adverse effect of motor inhibition and tonic syncope than that of typical neuroleptics cause. It may reduce the extrapyramidal side effects through the action at 5HT and dopamine antagonist, and extend its therapeutic action to negative symptoms and emotional symptoms of schizophrenia. Risperidone reaches peak plasma levels quickly regardless of whether it is administered as a liquid or pill and is metabolized quickly. However, the active metabolite, 9-hydroxy-risperidone, which has similar pharmacodynamics to risperidone, lingers in the body for much longer, and has also been developed as an antipsychotic called paliperidone.

U.S. Pat. No. 4,804,663 describes a synthesis of risperidone. Risperidone may be prepared by condensation of the following two intermediates, 6-fluoro-3-(4-piperidinyl)-1, 2-benzisoxazole and 3-(2-chloroethyl)-6,7,8,9-tetrahydro-2-methyl-4H-pyrido[1,2-a]pyrimidin-4-one in dimethylformamide (DMF) in basic conditions (Na₂CO₃ or K₂CO₃) with catalytic amount of potassium iodide (KI).

Current dosage forms of risperidone include a tablet in 0.25, 0.5, 1, 2, 3 and 4 mg sizes, as an oral solution (30ml, 1 mg/ml), and as a 12.5 mg, 25 mg, 37.5 mg and 50 mg ampoule Risperdal Consta, which is a depot injection administered once every two weeks. Risperdal Consta is slowly released from the injection site, facilitating use on sanctioned patients who are declining, or consenting patients and who may have disorganized thinking and cannot remember to take their daily doses. Doses range from 12.5 to 50 mg given as an intramuscular injection once every two weeks. It is also available as a wafer known in the United States and Canada as Risperdal M-Tabs and elsewhere as Risperdal Quicklets.

Clozapine is also classified as an atypical antipsychotic drug because its profile of binding to serotonergic as well as dopamine receptors. In particular, clozapine interferes to a lower extent with the binding of dopamine at D₁, D₂, D₃ and D₅ receptors, and has a high affinity for the D₄ receptor, but it does not induce catalepsy nor inhibit apomorphine-induced stereotypy in animal models as is seen with 'conventional' neuroleptics. This evidence suggests clozapine is preferentially more active at limbic than at striatal dopamine receptors and may explain the relative freedom of clozapine from extrapyramidal side effects together with strong anticholinergic activity.

Clozapine (8-chloro-11-(4-methyl-1-piperazinyl-5H-dibenzo[b,e][1,4] diazepine) is a well-known compound having anti-psychotic activity. Details about this compound are disclosed in monograph 2448 of the 13th edition of the Merck Index.

Clozapine is currently used for the management of schizophrenic patients who fail to respond adequately to or are intolerant of standard drug treatment for schizophrenia. Clozapine is also used in the treatment of Parkinson related psychosis. Clozapine is available as oral tablets or as an oral suspension.

### b. Formulations

Controlled release refers to the release of an agent such as a drug from a composition or dosage form in which the agent is released according to a desired profile over an extended period of time. Controlled release profiles include, for example, sustained release, prolonged release, pulsatile release, and delayed release profiles. In contrast to immediate release compositions, controlled release compositions allow delivery of an agent to a subject over an extended period of time according to a predetermined profile. Such release rates can provide therapeutically effective levels of an agent for an extended period of time and thereby provide a longer period of pharmacologic or diagnostic response as compared to conventional rapid release dosage forms. Such longer periods of response provide for many inherent benefits that are not achieved with the corresponding short acting, immediate release preparations. For example, in the treatment of chronic pain, controlled release formulations are often highly preferred over conventional short-acting formulations.

Controlled release pharmaceutical compositions and dosage forms are designed to improve the delivery profile of agents, such as drugs, medicaments, active agents, diagnostic agents, or any substance to be internally administered to an animal, including humans. A controlled release composition is typically used to improve the effects of administered substances by optimizing the kinetics of delivery, thereby increasing bioavailability, convenience, and patient compliance, as well as minimizing side effects associated with inappropriate immediate release rates, such as a high initial release rate and, if undesired, uneven blood or tissue levels.

The term bioavailability is used to describe the degree to which a drug becomes available at the site(s) of action after administration. The degree and timing in which an agent such as a drug becomes available to the target site(s) after administration is determined by many factors, including the dosage form and various properties, e.g., dissolution rate of the drug. It is well known that some drug compositions suffer from poor bioavailability because of poor solubility of the active ingredient itself.

Numerous methods have been developed for enhancing the bioavailability of poorly soluble drugs. Particle size reduction, such as nanoparticulate forms of the agent, is one such method since the dissolution rate of a compound is related to the particle size. Nanoparticulate compositions comprise poorly water-soluble drug or agent particles having an extremely small particle size, i.e., less than one micron. With a decrease in particle size, and a consequent increase in surface area, a composition tends to be rapidly dissolved and absorbed following administration. For certain formulations, this characteristic can be highly desirable, as described, for example, in U.S. Pat. Nos. 5,145,684, 5,510,118, 5,534,270, and 4,826,689. However, rapid dissolution is contrary to the goal of controlled release. Known controlled release formulations do not present a solution to this problem.

Exemplary controlled release formulations known in the art include specially coated pellets, microparticles, implants, tablets, minitabs, and capsules in which a controlled release of a drug is brought about, for example, through selective breakdown of the coating of the preparation, through release through the coating, through compounding with a special matrix to affect the release of a drug, or through a combination of these techniques. Some controlled release formulations provide for pulsatile release of a single dose of an active compound at predetermined periods after administration.

U.S. Pat. No. 5,110,605 to Acharya et al. refers to a calcium polycarbophil-alginate controlled release composition. U.S. Pat. No. 5,215,758 to Krishnamurthy et al. refers to a controlled release suppository composition of sodium alginate and calcium salt. U.S. Pat. No. 5,811,388 to Friend et al. refers to a solid alginate-based formulation including alginate, a water-swellable polymer, and a digestible hydrocarbon derivative for providing controlled release of orally administered compounds. WO 91/13612 refers to the sustained release of pharmaceuticals using compositions in which the drug is complexed with an ion-exchange resin. U.S. Pat. No. 5,811,425 to Woods et al. refers to injectable depot forms of controlled release drugs made by forming microencapsule matrices of the drug in biodegradable polymers, liposomes, or microemulsions compatible with body tissues. U.S. Pat. No. 5,811,422 to Lam et al. refers to controlled release compositions obtained by coupling a class of drugs to biodegradable polymers, such as polylactic acid, polyglycolic acid, copolymers of polylactic and polyglycolic acid, polyepsilon caprolactone, polyhydroxy butyric acid, etc. U.S. Pat. No. 5,811,404 to De Frees et al. refers to the use of liposomes having prolonged circulation half-lives to provide for the sustained release of drug compositions.

Any suitable method can be used to generate aerosol formulations of the present disclosure. For instance, one method involves heating a composition comprising drugs with affinity for both serotonergic 5-HT₂ and dopaminergic receptors such as the D₂ receptor to form a vapor, followed by cooling of the vapor such that it condenses to provide a risperidone comprising aerosol (condensation aerosol). The composition is heated in one of four forms: as pure active compound of risperidone, as a mixture of active compound and a pharmaceutically acceptable excipient; as a salt form of the pure active compound; and, as a mixture of active compound salt form and a pharmaceutically acceptable excipient.

Salt forms of drugs with affinity for both serotonergic 5-HT₂ and dopaminergic receptors, such as the D₂ receptor may be obtained from the corresponding free base. A variety of pharmaceutically acceptable salts are suitable for aerosolization. Such salts include, but are not limited to the following: hydrochloric acid, hydrobromic acid, acetic acid, maleic acid, formic acid, and fumaric acid salts.

Pharmaceutically acceptable excipients may be volatile or nonvolatile. Volatile excipients, when heated, are concurrently volatilized, aerosolized and inhaled with the antipsychotic. Classes of such excipients are known in the art and include, without limitation, gaseous, supercritical fluid, liquid and solid solvents. The following is a list of exemplary carriers within the classes: water; terpenes, such as menthol; alcohols, such as ethanol, propylene glycol, glycerol and other similar alcohols; dimethylformamide; dimethylacetamide; wax; supercritical carbon dioxide; dry ice; and mixtures thereof.

The heating of drugs with affinity for both serotonergic 5-HT₂ and dopaminergic receptors, such as the D₂ receptor composition can be performed using any suitable method. Exemplary methods by which heat can be generated include the following: passage of current through an electrical resistance element; absorption of electromagnetic radiation, such as microwave or laser light; and, exothermic chemical reactions, such as exothermic salvation, hydration of pyrophoric materials and oxidation of combustible materials.

Aerosols containing drugs with affinity for both serotonergic 5-HT₂ and dopaminergic receptors such as the D₂ receptor can be delivered to a mammal using an inhalation device. Where the aerosol is a condensation aerosol, the device has at least three elements: an element for heating an antipsychotic containing composition to form a vapor; an element allowing the vapor to cool, thereby providing a condensation aerosol; and, an element permitting the mammal to inhale the aerosol. Various suitable heating methods are described above. The element that allows cooling is, in it simplest form, an inert passageway linking the heating means to the inhalation means. The element permitting inhalation is an aerosol exit portal that forms a connection between the cooling element and the mammal's respiratory system.

The dosage amount of drugs with affinity for both serotonergic 5-HT₂ and dopaminergic receptors, such as the D₂ receptor in aerosol form is generally no greater than twice the standard dose of the drug given orally. A typical dosage of an antipsychotic containing aerosol is either administered as a single inhalation or as a series of inhalations taken within an hour or less (dosage equals sum of inhaled amounts). Where the drug is administered as a series of inhalations, a different amount may be delivered in each inhalation.

One can determine the appropriate dose of drugs with affinity for both serotonergic 5-HT₂ and dopaminergic receptors, such as the D₂ receptor containing aerosols to treat multiple sclerosis using methods such as animal experiments and a dose-finding (Phase I/II) clinical trial. One animal experiment involves measuring plasma concentrations of drug in an animal after exposure to the aerosol. Mammals such as dogs or primates are typically used in such studies, since their respiratory systems are similar to that of a human. Initial dose levels for testing in humans is generally less than or equal to the dose in the mammal model that resulted in plasma drug levels associated with a therapeutic effect in humans. Dose escalation in humans is then performed, until either an optimal therapeutic response is obtained or a dose-limiting toxicity is encountered.

### c. Dosage

The actual dosage amount of a composition for delivery of drugs which are known to be D₂ antagonists and 5HT antagonists such as risperidone, paliperidone or clozapine that is administered to a patient or subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In the present invention, pharmaceutical compositions comprise from 1 milligram/kg/body weight to 5 milligram/kg/body weight of clozapine per administration.

An effective amount of the therapeutic composition is determined based on the intended goal. The term "unit dose" or "dosage" refers to physically discrete units suitable for use in a subject, each unit containing a predetermined-quantity of the therapeutic composition calculated to produce the desired responses discussed above in association with its administration (i.e., the appropriate route and treatment regimen). The quantity to be administered, both according to number of treatments and unit dose, depends on the protection or effect desired.

Precise amounts of the drugs, each alone or in combination also depend on the judgment of the practitioner and are peculiar to each individual. Factors affecting the dose include the physical and clinical state of the patient, the route of administration, the intended goal of treatment (e.g., alleviation of symptoms versus cure) and the potency, stability and toxicity of the particular therapeutic substance.

Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### EXAMPLES

The following examples are included to demonstrate preferred embodiments of the disclosure. It should be appreciated by those of skill in the art that the techniques disclosed in the examples which follow represent techniques discovered by the inventors to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the scope of the disclosure, as defined in the appended claims. The following examples are offered by way of illustration and not by way of limitation.

Previously it was demonstrated that treating C57BL/6 mice with risperidone does not alter the onset or incidence of EAE. However, risperidone shows a dose-dependent reduction in the severity of the disease. This reduction in severity correlates to an increase in T regulatory cells (Tregs) chronically and a decrease in auto-reactive T cell responses during the acute stage.

### Example 1

### Determination of Optimum Drug and Treatment Regime in the C57BL/6 EAE Model

Preliminary comparative studies have shown that treating animals daily with risperidone from the time of immunization results in a dose-dependent reduction in the severity of EAE disease. In contrast, drug treatment does not alter the time of onset or the incidence of EAE. Analysis of the cellular profile of mice indicates that immunized mice receiving risperidone have increased numbers of splenic T regulatory cells whereas helper T cells were unaffected during the chronic stage of disease. Additionally, the number of neutrophils in the spleen was significantly reduced which together with the increase in T regulatory cells suggests that alterations in these cellular subsets may contribute to disease protection. Antigen-specific IL-17 and IL-10 but not IFN-γ production was reduced in treated mice during the acute stage suggesting a possible effect on the priming of auto-reactive T cells in these mice. Examples disclosed herein test the prevention of immune-mediated damage caused by immune dysfunction in multiple sclerosis with the use of risperidone.

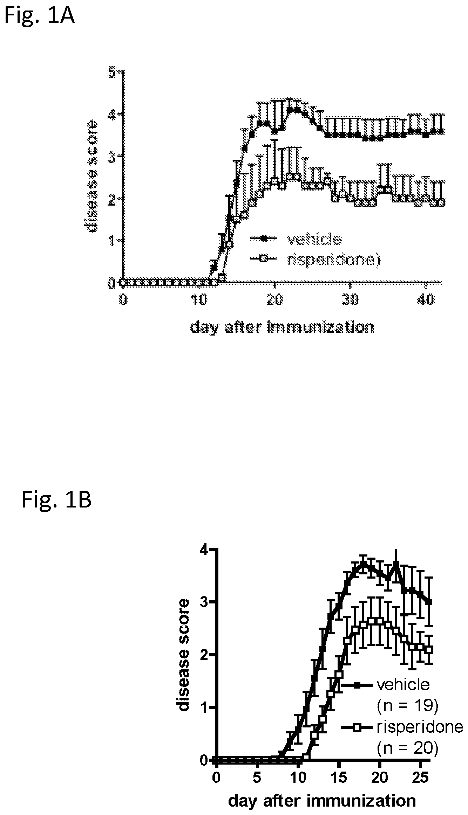

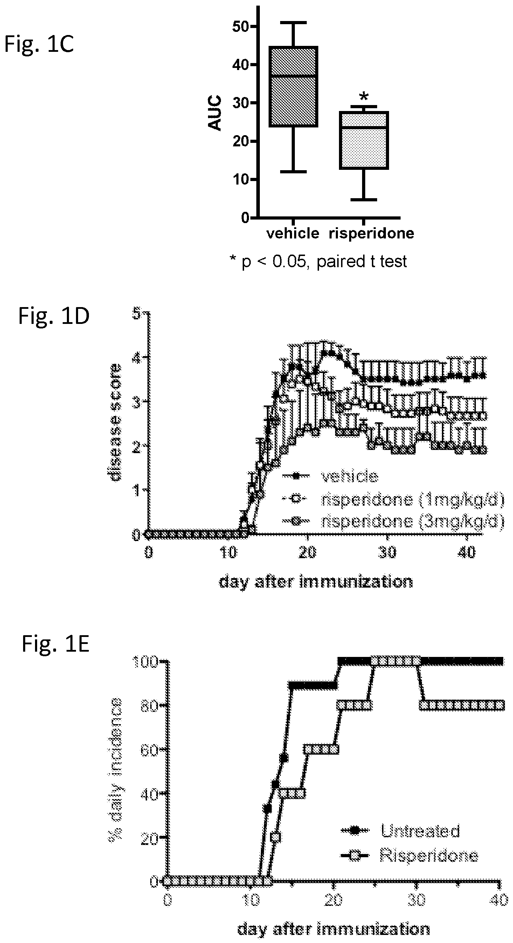

In these preliminary comparative studies risperidone was given in the drinking water at a calculated dose of 1 or 3 mg/kg/day. More specifically, C57BL/6 mice were administered this risperidone formulation starting from one day before immunization to induce EAE (FIG. 1A-E). The results indicate that this treatment shows significantly reduced disease. The reduction in disease is reproducible and leads to a stable reduction in average disease score during the chronic stage. Interestingly, risperidone treatment at 3 mg/kg/day is more effective in this model compared to 1 mg/kg/day. These doses had no detrimental effects on the mice (either immunized or unimmunized mice) aside from slight weight gain as has been reported by Pajonk, 2004. Previous work has shown that mice treated daily with doses up to 10 mg/kg/day for 18 months showed no toxicity Pajonk, 2004. Moreover, that study was unable to identify a maximum tolerated dose in mice suggesting that risperidone has a large therapeutic window.

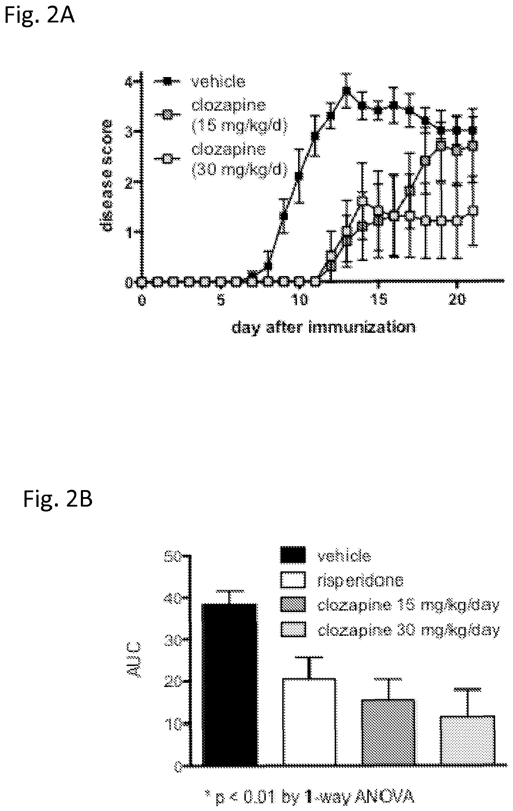

In a similar study, clozapine was administered to C57BL/6 mice in the drinking water at a calculated dose of 15 or 30 mg/kg/day starting from one day before immunization to induce EAE. The results indicate that this treatment shows significantly reduced disease (Fig. 2A). Interestingly, clozapine treatment at 30 mg/kg/day is more effective in this model compared to 15 mg/kg/day. The reduction in disease is reproducible and leads to a stable reduction in average disease score during the chronic stage. This reduction is similar to that achieved with risperidone at 3 mg/kg/day (Fig. 2B).

For drugs to be practical in treating multiple sclerosis, they must be effective at reducing on-going disease. In order to determine if risperidone can reduce disease severity after the onset of disease and/or enhance neuronal repair, one would determine if treating C57BL/6 mice with risperidone just after the onset of disease or during the chronic stage of EAE is effective. Treatment for mice treated after the onset of disease can begin on the day that they have a score of > 1.0 (loss of tail tonicity). Commonly this time period is between 13-20 days post immunization with myelin oligodendrocyte glycoprotein (50 µg/mouse) in complete Freund's adjuvant and injected i.p with pertussis toxin (200 ng/mouse) on days 0 and 2. Treatment during the "chronic" stage (when disease scores remain stable) may begin 30 days post immunization with myelin oligodendrocyte glycoprotein. For all of these studies, the optimal dose of risperidone may be used as determined by the experiments described above. Additionally, the C57BL/6 EAE mouse model can be used, which is thought to represent a progressive form of MS as reported by Steinman, 1999. This model is well established and has the added benefit of having many different immunological reagents available for use in the C57BL/6 system.

### Example 2

### Determination of Mechanism of Action of drugs with affinity for both serotonergic 5-HT₂ and dopaminergic receptors such as the D₂ receptor in vivo

Identification of the mechanism by which risperidone (comparative) reduces EAE disease can be performed by immunizing and treating C57BL/6 mice with the optimal treatment regimen as determined in Example 1 and the effect of risperidone on immune responses and CNS inflammation can be investigated. The effect of drug treatment on antigen-specific immune responses can be determined at 8 (pre-onset), 16 (peak disease), and 30 (chronic) days post immunization by isolating and culturing draining lymph node (LN) cells and splenocytes from untreated and risperidone-treated, immunized or unimmunized mice. Cytokine production in response to myelin oligodendrocyte glycoprotein antigen (MOG) can be assessed by cytokine bead assay, which measures 10 cytokines simultaneously, or ELISA, and proliferation can be measured by ³H thymidine uptake. Cellular profiles in the spleen and lymph node cells can be determined by flow cytometric analysis and can include markers for helper T cells, T regulatory cells, and macrophages.

The effects of treatment using risperidone on CNS inflammation and repair/regeneration can allow for a greater understanding of how risperidone treatment reduces CNS disease. For these studies two complementary approaches may be taken. First, a quantitative assessment of CNS infiltration by flow cytometry of CD45+ (i.e. haematopoietic) cells in the spinal cord and brains of treated or untreated mice is performed. This method allows a precise phenotypic analysis on infiltrating cells (i.e. subset markers and activation markers). Second, to determine the location of these cells within the tissue and to determine lesion characteristics (i.e. size, number, location), brain and spinal cords may be fixed in zinc salts or paraformaldehyde and tissue sections stained with haematoxylin & eosin (H&E) or Luxol Fast Blue. The H&E stained sections can allow for the determination of lesion number, size and location whereas the Luxol Fast Blue will reveal the level of demyelination in untreated and treated immunized mice at various times after immunization. In particular, these methods can be used to compare myelination at peak disease (16 days post infection) and during the chronic stage (30+ days post infection).

At least two approaches may be used to provide insight into mechanisms of action of risperidone in the EAE model. First, the studies demonstrate that risperidone enhances or expands the suppressive effects of T regulatory cells. To confirm, T regulatory cells can be neutralized by anti-CD25 antibody. A method of neutralizing T regulatory cells with anti-CD25 antibody has been reported by Quinn *et al.,* 2006. Accordingly, to confirm that disease reduction is mediated by T regulatory cells, neutralizing their activity will abolish the protective drug effect. The other approach that may be used to provide insight into the mechanisms of action of risperidone in the EAE model may reveal that the initial activation of self-reactive T cells is altered by risperidone. To confirm, CD4 T cells from untreated or treated immunized mice are adoptively transferred to naive animals and the induction of disease assessed. This method has been reported by Kuchroo *et al.,* 1992. If T cells from untreated animals but not drug-treated animals induce EAE, then it is likely that risperidone is altering T cell priming but not necessarily the later stages of disease (e.g. CNS infiltration and damage). Overall, these two approaches can be used to determine if helper T cells or Tregs are involved.

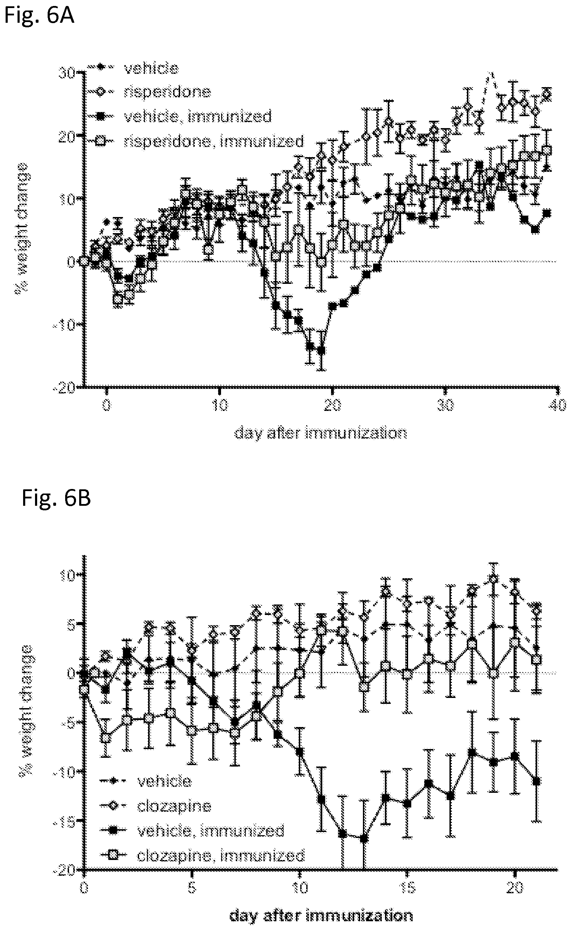

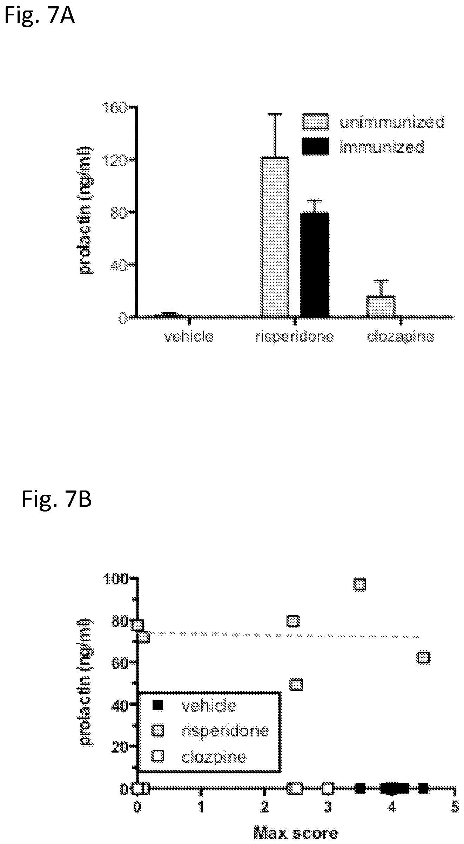

Further studies regarding weight gain and loss herein demonstrate that the combination of risperidone (comparative) or clozapine treatment promotes weight gain in normal mice, and in mice immunized with MOG (myelin oligodendrocyte glycoprotein) peptide, risperidone or clozapine reduce the weight loss that is commonly associated with EAE (Fig. 6A-B). Additionally, treatment with risperidone alone demonstrated high levels of serum prolactin in both immunized and unimmunized mice (Fig. 7A). This was not observed with clozapine treatment (Fig. 7A). However, the level of serum prolactin does not correlate to disease severity (i.e. those with a higher level of prolactin do not have reduced disease; Fig. 7B).

### Example 3

### Investigation of the Direct Effect of Risperidone on Macrophages and Microglia

The direct cellular effects of risperidone (comparative) can be determined by assessing how this drug alters cellular responses after in vitro culture and stimulation of primary macrophages and microglia as risperidone is thought to alter activation of these cells. Identification of how risperidone alters pro-inflammatory and/or anti-inflammatory immune responses is necessary. This can be achieved by isolating and culturing primary bone marrow-derived macrophages or microglia as described by Tierney *et al.,* 2008 and Saura et al., 2003. Briefly, after overnight activation in the presence or absence of IFN-γ (200 U/ml), macrophages and microglia are cultured with pro-inflammatory products (e.g. lipopolysaccharide) or anti-inflammatory products (e.g. IL-4) without risperidone or in increasing doses of risperidone (1-100 µg/ml). Glatiramer acetate (100 µg/ml) can be used as a positive control as it has been shown to reduce pro-inflammatory (i.e. IL-12) cytokine production by LPS-stimulated macrophages (La Flamme, unpublished results). At various time points after the addition of stimuli, culture supernatants will be collected for analysis of cytokine production (esp. IL-12, TNF-α, MCP-1, IL-6, and IL-10) as demonstrated by Keating *et al.,* 2009. Changes in the expression of activation markers (esp. MHC class II, CD40, CD80, CD86, and PD-L1) are also determined on macrophages and microglia by flow cytometry as described by Tierney et al., 2008.

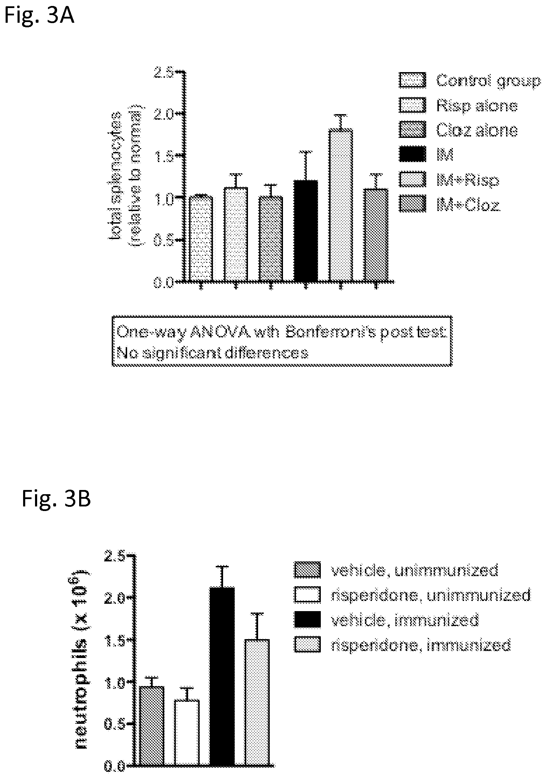

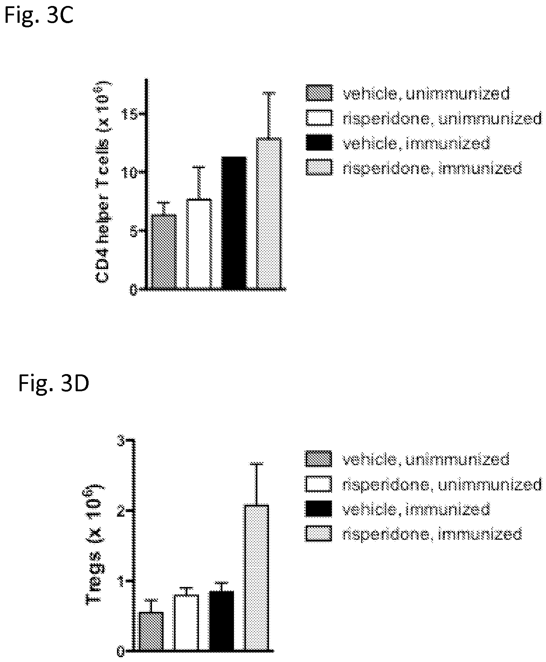

Studies herein indicate that risperidone (comparatiave) or clozapine treatment does not result in overall loss of immune cells in the spleen during peak disease (day 21) (Fig. 3A). Similar overall numbers of immune cells are present after risperidone or clozapine treatment of unimmunized and immunized mice compared to vehicle treated controls. Studies further indicate that risperidone does however reduce the number of neutrophils (Gr1+ cells) in the spleen during the chronic stage of disease (FIG. 3B). Risperidone does not alter the number of helper T cells (CD4+CD25-) in the spleen during the chronic stage of disease (Fig. 3C). However, risperidone does enhance in the number of regulatory T cells (CD4+CD25+ Treg) in the spleen during this stage of disease (FIG. 3D).

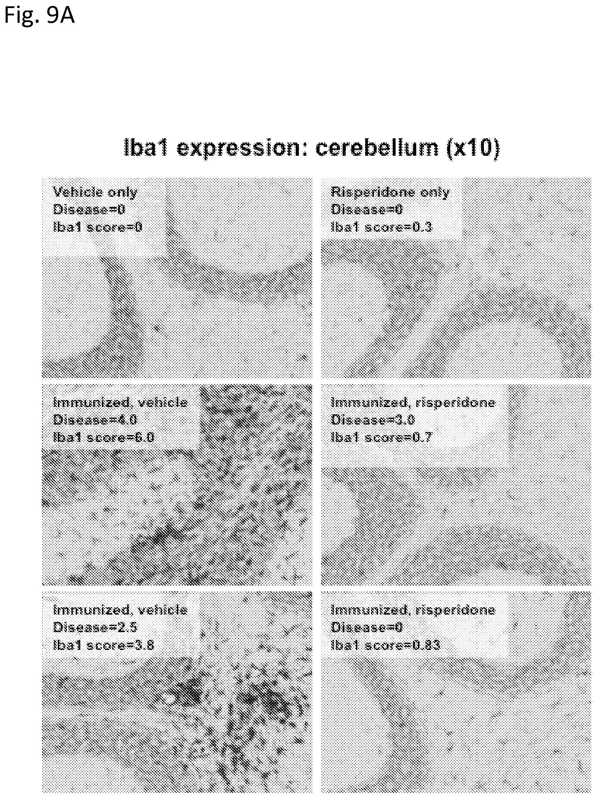

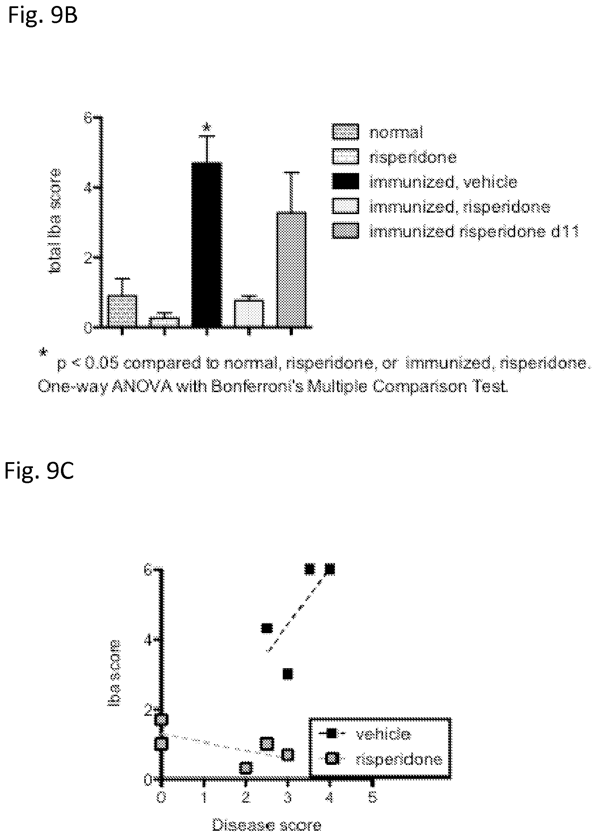

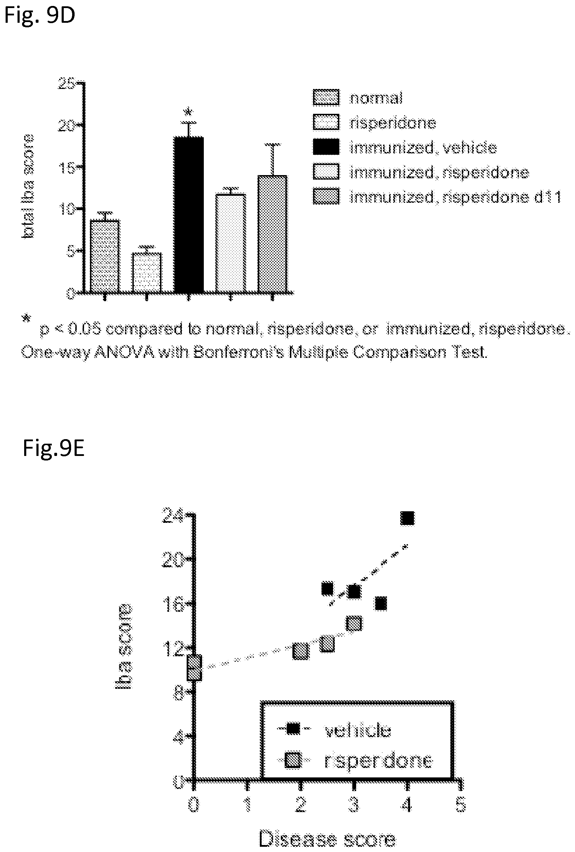

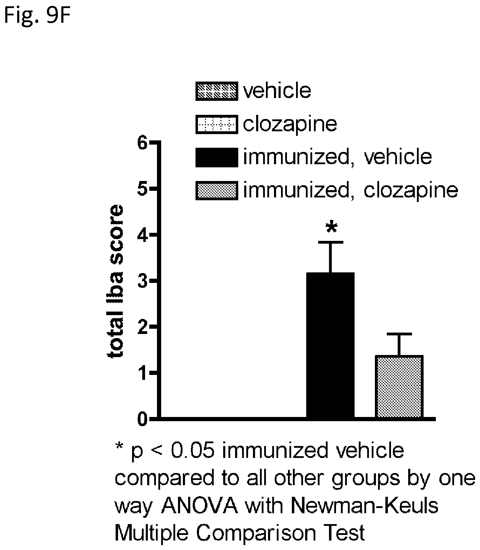

Studies were performed to assess microglial reactivity via the expression of Iba-1 (FIG. 9A-F). The results herein indicate that the expression of Iba-1 is greatly enhanced during EAE in vehicle treated mice. However, this high microglial reactivity is greatly reduced in immunized mice by risperidone (comparative) or clozapine treatment. Treatment with risperidone alone starting at day 11 post-immunization also reduces microglial reactivity on day 15. When mice are treated with either risperidone (comparative) or clozapine, the effects are seen throughout the brain as assessed in the cerebellum, brain stem, hippocampus, and olfactory bulb, although the effects are most marked in the cerebellum.

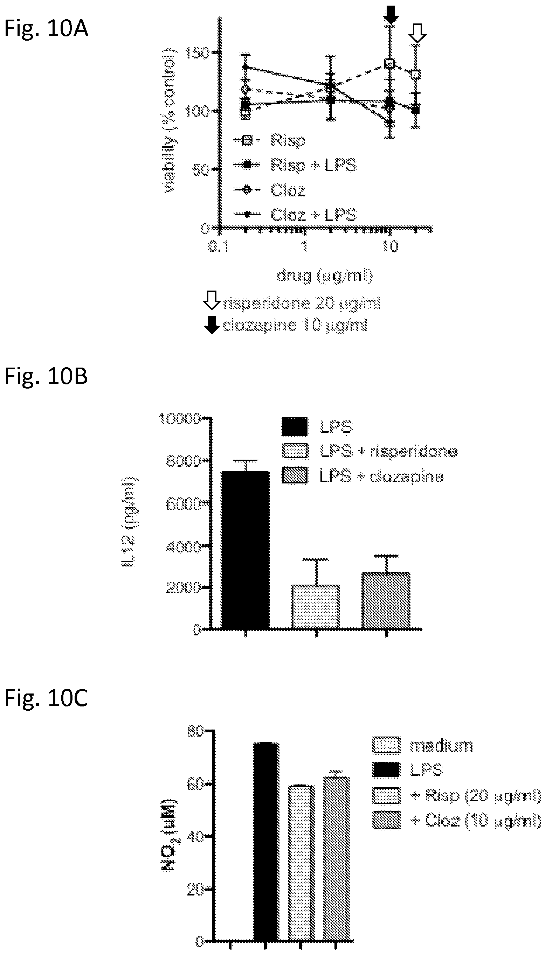

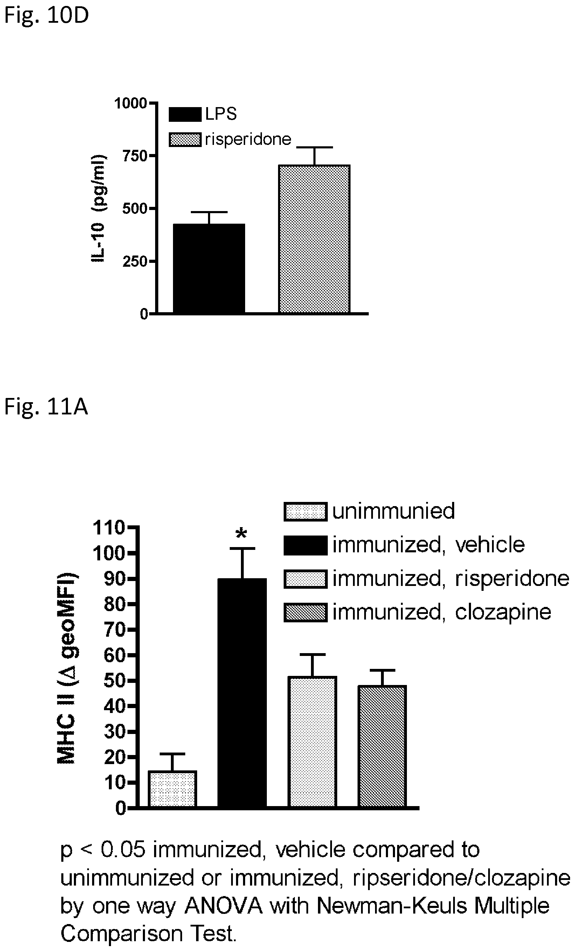

Studies were conducted herein to evaluate the regulation of macrophage proinflammatory responses in vitro. Bone marrow derived macrophages were administered 20 µg/ml risperidone or 10 µg/ml clozapine *in vitro* in the presence or absence of LPS. These treated macrophages were fully viable following treatment (FIG. 10A). The risperidone (comparative) or clozapine treated macrophages were observed to produce less of the pro-inflammatory mediators IL-12 and NO in response to LPS than vehicle-treated macrophages (FIG. 10B). However, the risperidone treated macrophages were observed to produce more of the anti-inflammatory cytokine IL-10 in response to LPS than vehicle-treated macrophages (FIG. 10C-D).

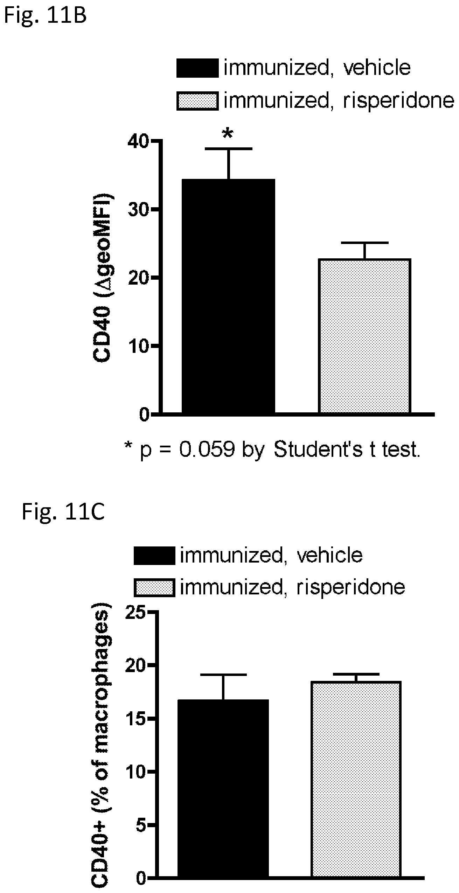

Studies were also conducted herein to evaluate the regulation of macrophage proinflammatory responses in vivo. Splenic macrophages show reduced MHC class II expression in risperidone-treated (comparative), immunized mice compared to vehicle-treated immunized mice during peak disease suggesting that splenic macrophages from risperidone-treated mice are less activated during EAE than after vehicle-treatment (FIG. 11A). Additionally, splenic macrophages show reduced CD40 expression in risperidone-treated, immunized mice compared to vehicle-treated immunized mice during peak disease suggesting that splenic macrophages from risperidone-treated mice are less activated during EAE than after vehicle-treatment (FIG. 11B). Despite expressing lower levels of CD40, the number of CD40-expressing splenic macrophages at peak disease is not reduced by risperidone treatment during EAE supporting the idea that risperidone reduces activation but does not lead to cell death/depletion (FIG. 11C).

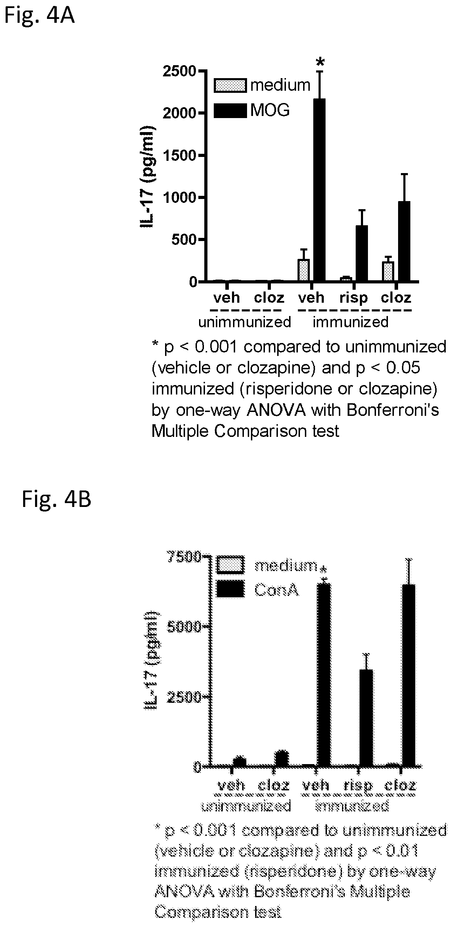

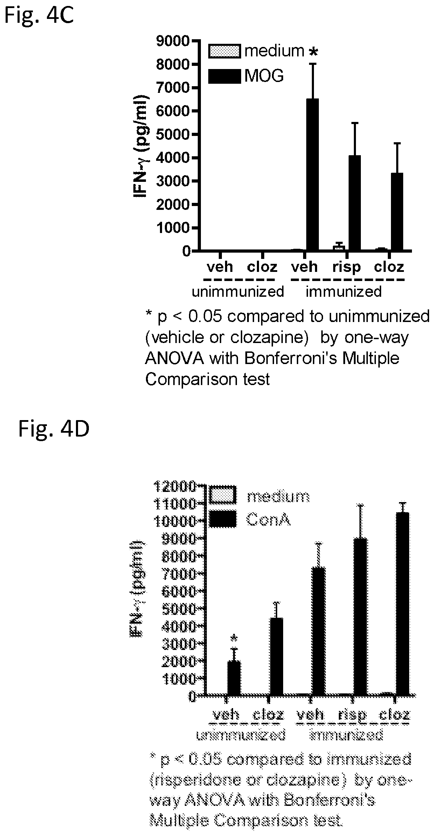

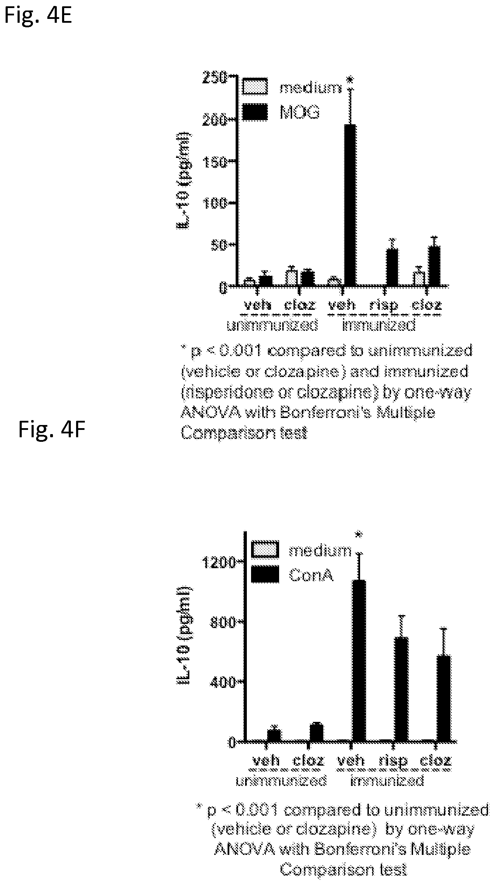

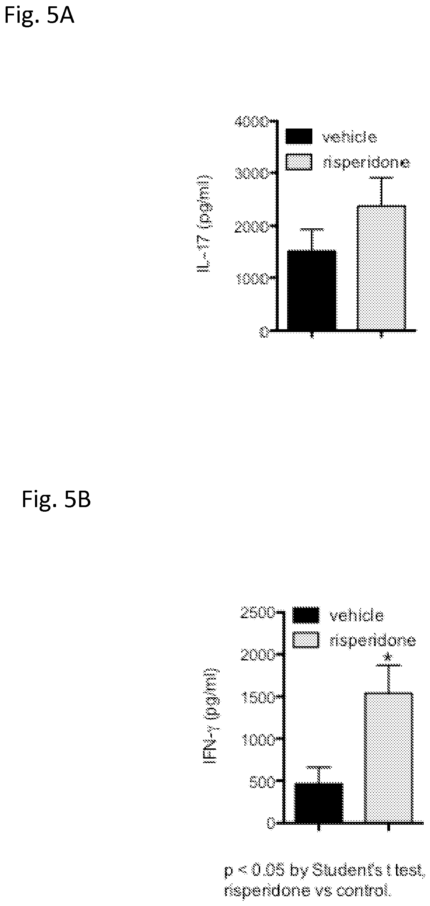

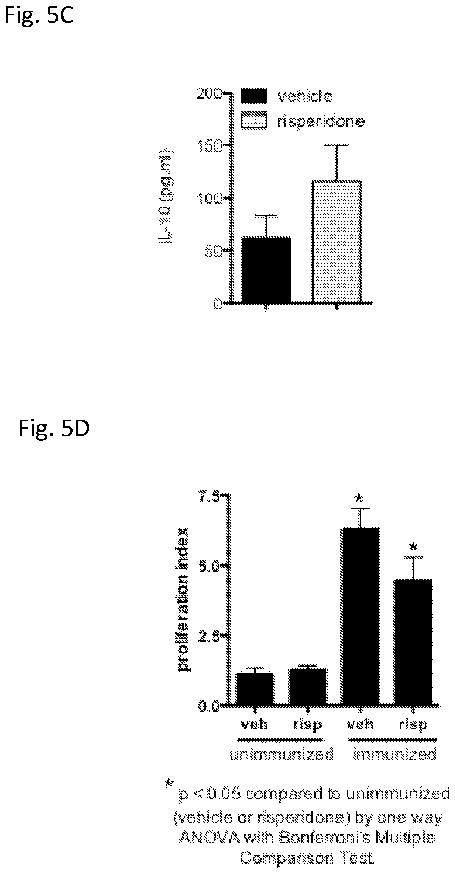

Additional studies were conducted herein on isolated cells treated in vitro and mice treated in vivo with risperidone or with clozapine to evaluate both autoimmune response and general immune response during peak disease at 21 days post immunization (FIG. 4A-F) and 40 days post immunization (FIG. 5A-5D). The findings herein indicate that risperidone and clozapine treatment significantly reduce the production of IL-17 and IL-10 in response to MOG (myelin oligodendrocyte glycoprotein) antigen during peak disease, but treatment with these risperidone and clozapine treatment does not alter the production of IFN-γ. In contrast, when risperidone and clozapine mice were treated with a polyclonal stimulus (ConA), no alteration in the production of IL-10 or INF-γ was observed. Risperidone alone however, does enhance MOG specific IFN-γ production during the chronic stage of the disease, but has no effect on MOG-specific proliferation, IL-10 production, or IL-17 production.

### Experiment 4

### Disease Severity and Incidence in the BALB/c EAE Model with Risperidone Treatment

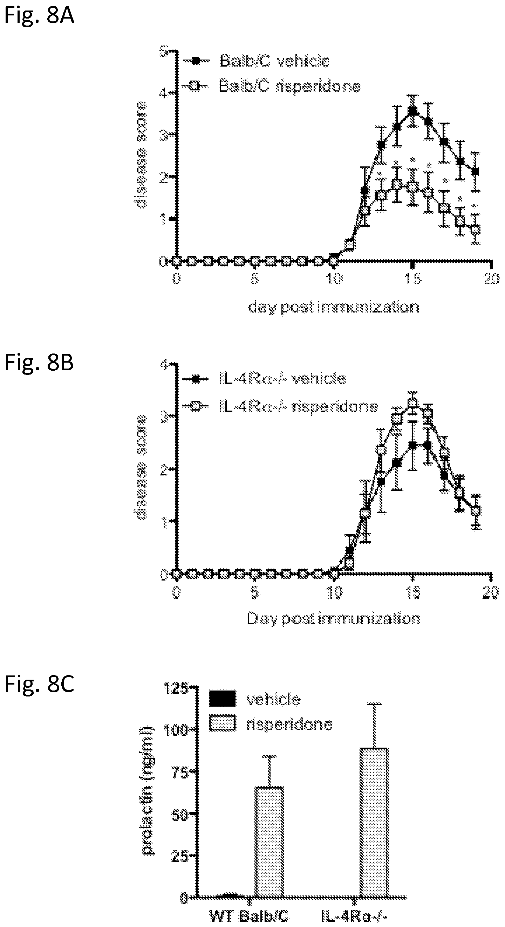

Mice were treated with risperidone (comparative) orally beginning one day before immunization with PLP in complete Freund's adjuvant to induce EAE (n = 11 per group). Both vehicle and risperidone treated, immunized mice had similar incidences and onset of disease, but risperidone treated mice has a significantly reduced disease severity (Fig. 8A). BALB/c mice are considered more resistant to EAE but develop brain pathology, which is more similar to that seen in MS patients.

It was discovered that BALB/c mice deficient in IL-4Rα do not experience a reduction in disease symptoms even after risperidone treatment, suggesting IL-4Rα is involved in the reduction of disease by risperidone (FIG. 8B). Additionally, both BALB/c and IL-4Rα mice develop hyperprolactinemia after risperidone treatment and the level of prolactin in the serum does not correlate to disease score (FIG. 8C).

Example embodiments have been described hereinabove regarding compositions for the treatment of multiple sclerosis. Various modifications to and departures from the disclosed example embodiments will occur to those having ordinary skill in the art. The subject matter that is intended to be within the scope of this disclosure is set forth in the appended claims.

## Claims

1. A pharmaceutical formulation for use in the treatment of at least one symptom of multiple sclerosis, wherein the formulation comprises a drug selected from clozapine, **characterised in that** the dose of the drug is in the range of 1 mg/kg/body to 5 mg/kg/body.

2. A pharmaceutical formulation for use according to claim 1, wherein the pharmaceutical formulation is formulated as an oral formulation, an epidural formulation, a transdermal formulation, a subcutaneous formulation an intravenous formulation, an intramuscular formulation, an intrathecal formulation, an intraperitoneal formulation or an aerosol formulation.

3. A pharmaceutical formulation for use according to claim 1, wherein the pharmaceutical formulation further comprises risperidone.

4. A pharmaceutical formulation for use according to claim 1, wherein the pharmaceutical formulation further comprises paliperidone.

5. A pharmaceutical formulation for use according to claim 1, wherein the pharmaceutical formulation further comprises risperidone and paliperidone.

6. The pharmaceutical formulation for use according to claim 1, wherein the multiple sclerosis symptom is selected from the group consisting of incontinence, loss of memory, paralysis, loss of coordination, fatigue, pain, optic neuritis, spasticity, transverse myelitis, tremor or a combination thereof.

## Patentansprüche

1. Pharmazeutische Formulierung zur Verwendung bei der Behandlung mindestens eines Symptoms von Multipler Sklerose, wobei die Formulierung ein Arzneimittel ausgewählt aus Clozapin umfasst, **dadurch gekennzeichnet, dass** die Dosis des Arzneimittels im Bereich von 1 mg/kg/Körper bis 5 mg/kg/Körper liegt.

2. Pharmazeutische Formulierung zur Verwendung nach Anspruch 1, wobei die pharmazeutische Formulierung als eine orale Formulierung, eine epidurale Formulierung, eine transdermale Formulierung, eine subkutane Formulierung, eine intravenöse Formulierung, eine intramuskuläre Formulierung, eine intrathekale Formulierung, eine intraperitoneale Formulierung oder eine Aerosol-Formulierung formuliert ist.

3. Pharmazeutische Formulierung zur Verwendung nach Anspruch 1, wobei die pharmazeutische Formulierung ferner Risperidon umfasst.

4. Pharmazeutische Formulierung zur Verwendung nach Anspruch 1, wobei die pharmazeutische Formulierung ferner Paliperidon umfasst.

5. Pharmazeutische Formulierung zur Verwendung nach Anspruch 1, wobei die pharmazeutische Formulierung ferner Risperidon und Paliperidon umfasst.

6. Pharmazeutische Formulierung zur Verwendung nach Anspruch 1, wobei das Symptom der Multiplen Sklerose ausgewählt ist aus der Gruppe bestehend aus Inkontinenz, Gedächtnisverlust, Paralyse, Verlust der Koordination, Ermüdung, Schmerz, Sehnerventzündung, spastischer Lähmung, Querschnittsmyelitis, Tremor oder einer Kombination davon.

## Revendications

1. Formulation pharmaceutique destinée à être utilisée dans le traitement d'au moins un symptôme de sclérose en plaques, dans laquelle la formulation comprend un médicament choisi parmi la clozapine, **caractérisée en ce que** la dose du médicament se situe dans la plage de 1 mg/kg/poids corporel à 5 mg/kg/poids corporel.

2. Formulation pharmaceutique destinée à être utilisée selon la revendication 1, dans laquelle la formulation pharmaceutique est formulée sous forme de formulation orale, formulation épidurale, formulation transdermique, formulation sous-cutanée, formulation intraveineuse, formulation intramusculaire, formulation intrathécale, formulation intrapéritonéale ou formulation en aérosol.

3. Formulation pharmaceutique destinée à être utilisée selon la revendication 1, dans laquelle la formulation pharmaceutique comprend, en outre, de la rispéridone.

4. Formulation pharmaceutique destinée à être utilisée selon la revendication 1, dans laquelle la formulation pharmaceutique comprend, en outre, de la palipéridone.

5. Formulation pharmaceutique destinée à être utilisée selon la revendication 1, dans laquelle la formulation pharmaceutique comprend, en outre, de la rispéridone et de la palipéridone.

6. Formulation pharmaceutique destinée à être utilisée selon la revendication 1, dans laquelle le symptôme de sclérose en plaques est choisi dans le groupe consistant en incontinence, perte de mémoire, paralysie, perte de coordination, fatigue, douleur, névrite optique, spasticité, myélite transverse, tremblement ou une combinaison de ceux-ci.
